# DEMANDE DE BREVET EUROPEEN

(11) **EP 2 574 313 A1**
(43) Date de publication de la demande: **03.04.2013**
(21) Numéro de dépôt: 12184777.6
(22) Date de dépôt: 18.09.2012
(51) Int. Cl.: A61F 2/40

(54) **Implant huméral de prothèse d'épaule**

(30) Priorité: 29.09.2011 FR 1158735
(71) Demandeur: FX Solutions, 01000 Bourg en Bresse (FR)
(72) Inventeur: Martin, Jean-Jacques, 01000 BOURG EN BRESSE (FR)
(74) Mandataire: Jeannet, Olivier

(57) **Abrégé**

Cet implant (1) comprend une pièce articulaire sphérique (2) et une pièce (3) d'ancrage à l'humérus (100), formant une cavité proximale de réception de cette pièce articulaire sphérique (2).

Selon l'invention,
- ladite cavité proximale est sous forme d'un siège sphérique (6a, 6b) apte à recevoir la pièce articulaire sphérique (2) avec calage de cette pièce articulaire par rapport à ce siège ; et
- la pièce d'ancrage (3) a une hauteur telle qu'elle est destinée à être implantée dans la partie épiphysaire ou métaphysaire de l'os (100), et comprend des reliefs (5) d'ancrage à l'os.

## Description

La présente invention concerne un implant huméral de prothèse d'épaule. Elle concerne également un ensemble d'éléments assemblables pour la constitution de cet implant huméral.

Un implant huméral de prothèse d'épaule comprend classiquement une tige médullaire destinée à être engagée dans le canal médullaire de l'humérus et une pièce articulaire proximale formant une surface articulaire, montée sur l'extrémité proximale de la tige médullaire. Cette dernière comprend une partie proximale évasée destinée à être engagée dans la partie métaphysaire de l'os, et une partie distale effilée, allant en se réduisant en section dans la direction distale, destinée à être engagée dans le canal médullaire de l'os. La pièce articulaire proximale est généralement apte à être montée sur la tige médullaire selon une pluralité de positions possibles, de manière à ce que le réglage de sa position permette de régler la position de l'humérus par rapport à l'omoplate.

Une telle tige médullaire a pour inconvénient de requérir un travail important d'ablation d'os pour sa mise en place, ce qui allonge l'intervention et rend cette intervention complexe. De plus, elle implique l'ablation d'une quantité non négligeable d'os, éventuellement sain. En outre, le réglage de la position de la pièce articulaire proximale par rapport à la tige médullaire peut être délicat à réaliser, et la nécessité d'opérer un tel réglage constitue une certaine contrainte.

La présente invention a pour objectif de remédier à cet inconvénient essentiel.

Il est par ailleurs connu d'aménager une cavité proximale glénoïde sur une tige médullaire et de concevoir la pièce articulaire sous la forme d'une sphère, cette pièce articulaire venant au contact ponctuellement avec la paroi de la tige médullaire délimitant ladite cavité proximale glénoïde et pouvant rouler sur celle-ci. En particulier, le document FR 2 917 963 décrit un implant huméral comprenant une pièce d'ancrage à l'humérus sous forme d'une tige médullaire allongée, et une pièce articulaire sphérique, ladite pièce d'ancrage à l'humérus formant une cavité proximale de réception de cette pièce articulaire sphérique.

Une telle prothèse supprime la nécessité d'opérer un réglage de la position de la pièce articulaire proximale par rapport à la tige médullaire mais ne remédie pas aux autres inconvénients précités. Elle a en outre pour inconvénient de constituer une articulation prothétique plutôt instable.

Par ailleurs, des qualités d'os très différentes peuvent se rencontrer d'un patient à un autre, et il n'existe pas à ce jour d'ensemble d'éléments assemblables permettant la constitution facile et rapide, au besoin en per opératoire, et sans changer substantiellement de technique de pose, d'un implant huméral de prothèse d'épaule.

La présente invention a également pour objectif de remédier à cette lacune.

L'implant concerné comprend, de manière connue en soi, une pièce articulaire sphérique et une pièce d'ancrage à l'humérus, formant une cavité proximale de réception de cette pièce articulaire sphérique.

Selon l'invention,
- ladite cavité proximale est sous forme d'un siège sphérique ajusté à la courbure de ladite pièce articulaire sphérique et délimité par un bord périphérique marqué, apte à recevoir cette pièce articulaire sphérique avec calage de cette pièce articulaire par rapport à ce siège, c'est-à-dire de telle sorte que le centre de la pièce articulaire sphérique n'ait pas de possibilité de se déplacer par rapport à la pièce d'ancrage lors du jeu articulaire de la prothèse ; et
- la pièce d'ancrage a une hauteur telle qu'elle est destinée à être implantée dans la partie épiphysaire ou métaphysaire de l'os, et comprend des reliefs d'ancrage à l'os.

Ainsi, à la différence des prothèses selon l'art antérieur, le centre de la pièce articulaire sphérique n'a pas de possibilité de se déplacer par rapport à la pièce d'ancrage lors du jeu articulaire de la prothèse. L'inventeur a pu constater que les prothèses connues, à pièces articulaires sphériques, impliquait en pratique l'utilisation d'un implant huméral à tige médullaire allongée pour résister aux efforts répétés exercés en porte à faux sur la paroi de cet implant huméral délimitant ladite cavité proximale glénoïde lors des roulements de la pièce articulaire sphérique sur cette paroi. Ces efforts s'exercent particulièrement lorsqu'une tension notable est créée sur la pièce articulaire sphérique pour contrôler l'instabilité de l'articulation.

Désirant solutionner ce problème, l'inventeur a été amené à concevoir un siège sphérique proximal marqué sur la pièce d'ancrage, recevant la pièce articulaire sphérique avec calage de cette pièce articulaire sphérique par rapport à ce siège. L'inventeur a découvert que cette absence de déplacement de la pièce articulaire sphérique par rapport à la pièce d'ancrage supprimait l'exercice d'efforts en porte à faux sur la pièce d'ancrage, ce qui lui a permis de concevoir une pièce d'ancrage à ancrage de faible profondeur (soit uniquement à ancrage épiphysaire ou métaphysaire, soit, selon la qualité de l'os, à ancrage essentiellement épiphysaire ou métaphysaire et également médullaire), remédiant ainsi aux inconvénients précités liés à la mise en place d'une tige médullaire.

La combinaison de la pièce articulaire sphérique et de la pièce d'ancrage à ancrage de faible profondeur s'avère en outre permettre un parfait fonctionnement de l'articulation reconstruite avec l'implant huméral selon l'invention.

Le siège sphérique pourrait être tel qu'il réalise le calage de la pièce articulaire sphérique sans possibilité de glissement de cette pièce articulaire sphérique par rapport à lui, ou avec une possibilité de glissement réduite. De préférence, toutefois, la paroi de la pièce d'ancrage délimitant ledit siège sphérique, ou la paroi de la pièce articulaire sphérique, ou les deux, sont lisses de manière à autoriser une possibilité de glissement de cette pièce articulaire par rapport à ce siège sphérique, par pivotement de cette pièce articulaire sphérique selon son centre.

Il sera également compris que par l'expression "possibilité de glissement" signifie que la pièce articulaire a une possibilité de mobilité autour de son centre, par rapport à la pièce d'ancrage, lors du jeu articulaire de la prothèse. Cette possibilité de glissement permet de limiter l'usure de cette pièce et est en outre favorable au jeu de l'articulation prothétique.

De préférence, la pièce d'ancrage a une forme conique ou tronconique.

Cette forme conique permet un parfait ancrage de la pièce d'ancrage à l'os. Notamment, la pièce d'ancrage peut être creuse intérieurement et être ainsi assimilable à un cornet, tronqué ou non au niveau de sa partie distale.

Les reliefs d'ancrage que comprend la pièce d'ancrage s'étendent avantageusement sur au moins une large portion de la périphérie de la pièce d'ancrage, de manière à présenter des surfaces étendues de prise d'appui contre l'os.

Ces reliefs d'ancrage peuvent être sous forme de collerettes étagées, à bords libres acérés, permettant une mise en place de la pièce d'ancrage dans l'os par impaction.

De préférence, la pièce d'ancrage comprend une cavité distale débouchant dans son extrémité distale, délimitée par la paroi périphérique distale de cette pièce d'ancrage.

Cette cavité est ainsi apte à former un réceptacle à greffon, permettant un ancrage à l'os de ladite pièce par croissance des cellules osseuses dans ce greffon.

Ladite paroi périphérique distale peut comprendre des ouvertures la traversant.

La croissance des cellules osseuses peut ainsi s'opérer au travers de ces ouvertures.

Ladite paroi périphérique distale peut comprendre au moins une fente la traversant et débouchant dans le bord distal de cette paroi périphérique distale.

Cette ou ces fentes permettent de conférer à la paroi périphérique distale une souplesse radiale, favorable à la réalisation d'un parfait ancrage distal de la pièce d'ancrage, et constituent également des ouvertures permettant la croissance des cellules osseuses dans le greffon.

Avantageusement, la pièce d'ancrage comprend, au niveau de sa partie distale, un moyen de liaison à une tige médullaire, notamment sous forme d'un plot fileté.

Cette pièce d'ancrage peut ainsi recevoir, lorsque la mauvaise qualité de l'os épiphysaire et métaphysaire le requiert, une telle une tige médullaire, cette dernière assurant un ancrage supplémentaire de cette pièce d'ancrage à l'humérus.

Lorsque la pièce d'ancrage comprend ladite cavité distale, ledit moyen de liaison peut notamment être aménagé au niveau de la paroi de la pièce d'ancrage délimitant le fond de cette cavité.

Ce moyen de liaison ne constitue ainsi pas une gêne à la mise en place de la pièce d'ancrage sur l'os lorsque ladite tige médullaire n'est pas utilisée.

L'ensemble d'éléments assemblables pour la constitution de l'implant huméral selon l'invention comprend :
- au moins une pièce articulaire sphérique telle que précitée ;
- au moins une pièce d'ancrage telle que précitée, comprenant, adjacent audit siège sphérique, un évidement proximal faiblement conique, apte à recevoir, avec coincement, une pièce à paroi périphérique faiblement conique correspondante, permettant le montage d'une pièce articulaire non sphérique formant une surface articulaire humérale prothétique ;
- au moins une pièce de montage telle que précitée ; et
- au moins une pièce articulaire non sphérique telle que précitée.

Cet ensemble d'éléments permet ainsi de constituer soit un implant huméral à pièce articulaire sphérique, soit, au moyen de l'insertion et du coincement de ladite pièce de montage dans ledit évidement proximal, un implant huméral à pièce articulaire formant une surface articulaire non sphérique.

De préférence, l'ensemble d'éléments comprend plusieurs pièces articulaires sphériques, de diamètres différents.

La pièce articulaire ayant le diamètre le plus adapté à l'articulation à reconstruire peut ainsi être choisie parmi la pluralité de pièces articulaires que comprend ledit ensemble d'éléments.

Le siège sphérique que comprend ladite pièce d'ancrage dudit ensemble d'éléments pourrait se réduire à une portée sphérique surmontant ledit évidement proximal faiblement conique ; de préférence toutefois, ce siège sphérique comprend une portée sphérique surmontant ledit évidement proximal faiblement conique, et une cavité en calotte sphérique aménagée dans le fond dudit évidement proximal faiblement conique, cette cavité en calotte sphérique étant aménagée selon une même sphère géométrique que ladite portée sphérique.

Le siège sphérique conserve ainsi une surface étendue de réception de la pièce articulaire sphérique, prévenant tout risque de marquage de la paroi de cette pièce susceptible de conduire à une perte ou une limitation de la possibilité de glissement de cette pièce articulaire par rapport à la pièce d'ancrage.

De préférence, l'ensemble d'éléments comprend plusieurs pièces d'ancrage, à savoir au moins une pièce d'ancrage de hauteur telle qu'elle est destinée à être ancrée uniquement à la zone épiphysaire de l'os et au moins une pièce d'ancrage de hauteur telle qu'elle est destinée à être ancrée à la zone épiphysaire et à la zone métaphysaire de l'os.

La pièce d'ancrage la plus adaptée à la qualité de l'os rencontrée peut ainsi être choisie parmi la pluralité de pièces d'ancrage que comprend ledit ensemble d'éléments.

L'invention sera bien comprise, et d'autres caractéristiques et avantages de celle-ci apparaîtront, en référence au dessin schématique annexé, représentant, à titre d'exemples non limitatifs, plusieurs formes de réalisation de l'implant huméral concerné.
La figure 1 est une vue en perspective de la pièce d'ancrage que comprend l'implant huméral selon une première forme de réalisation ;
la figure 2 est une vue de cette pièce d'ancrage de côté, en coupe longitudinale passant par un axe longitudinal central de cette pièce, et d'une pièce articulaire sphérique que comprend également l'implant huméral ;
la figure 3 est une vue antérieure d'une articulation de l'épaule reconstruite au moyen de l'implant huméral montré sur la figure 2 ; sur cette figure 2, la pièce articulaire sphérique coopère avec la surface glénoïde native de l'omoplate ;
la figure 4 est une vue en perspective de la pièce d'ancrage que comprend l'implant huméral selon une deuxième forme de réalisation ;
la figure 5 est une vue de cette pièce d'ancrage de côté, en coupe longitudinale passant par un axe longitudinal central de cette pièce, et d'une tige médullaire apte à assurer un ancrage supplémentaire de la pièce d'ancrage à l'os ;
la figure 6 est une vue en perspective de la pièce d'ancrage que comprend l'implant huméral selon une troisième forme de réalisation ;
la figure 7 est une vue antérieure d'une articulation de l'épaule reconstruite au moyen de l'implant huméral comprenant la pièce d'ancrage montrée sur la figure 6 et comprenant une tige telle que montrée sur la figure 5 et une pièce articulaire sphérique telle que montrée sur la figure 2 ; sur cette figure 7, la pièce articulaire sphérique coopère avec la surface glénoïde native de l'omoplate ;
la figure 8 est une vue de côté de l'implant huméral selon la figure 7, la pièce articulaire sphérique coopérant avec un implant de glène ;
la figure 9 est une vue de côté, en éclaté, de l'implant huméral selon une quatrième forme de réalisation, la pièce d'ancrage de cet implant étant vue en coupe passant par un axe longitudinal central de cette pièce ;
la figure 10 est une vue de côté de l'implant huméral selon la figure 9, après assemblage ;
la figure 11 est une vue de côté, en éclaté, de l'implant huméral selon une cinquième forme de réalisation, la pièce d'ancrage de cet implant étant vue en coupe passant par un axe longitudinal central de cette pièce ;
la figure 12 est une vue en perspective de la pièce d'ancrage que comprend l'implant huméral selon une sixième forme de réalisation ; et
la figure 13 est une vue de côté cette pièce d'ancrage, en coupe passant par un axe longitudinal central de cette pièce.

Par simplification, les parties ou éléments d'une forme de réalisation, qui se retrouvent de manière identique ou similaire sur une autre forme de réalisation, seront désignés par les mêmes références numériques et ne seront pas à nouveau décrits.

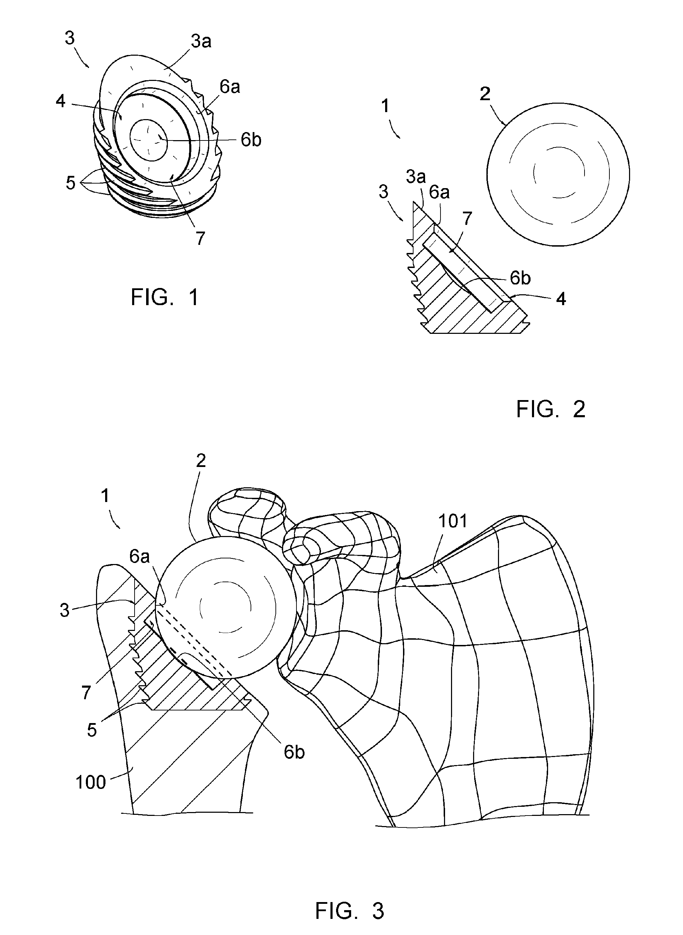

Les figures 2 et 3 représentent un implant huméral 1 de prothèse d'épaule, comprenant une pièce articulaire sphérique 2 et une pièce 3 d'ancrage à un humérus. Ainsi que le montre la figure 3, cet implant 1 permet de reconstruire une articulation de l'épaule, la pièce 3 étant ancrée à l'humérus 100 réséqué et la pièce 2 prenant place entre cette pièce 3 et la surface glénoïde native que forme l'omoplate 101.

La pièce articulaire sphérique 2 est formée par une boule en matériau biocompatible permettant un glissement, notamment en polyéthylène à haute densité ou en PEEK (polyétheréthercétone).

La pièce 3 est en métal biocompatible, notamment en titane. Comme le montrent les figures 1 à 3, elle est de forme conique, a une hauteur telle qu'elle est destinée à être implantée essentiellement dans la partie épiphysaire de l'os 100, présente une face proximale 3a inclinée dans laquelle est formée une cavité proximale 4 de réception de la pièce articulaire sphérique 2, et comprend des reliefs 5 d'ancrage à l'humérus 100.

La cavité proximale 4 forme un siège sphérique 6a, 6b et un évidement proximal 7 à paroi périphérique faiblement conique, c'est-à-dire dont la pente est de l'ordre de deux à sept degrés environ.

Le siège sphérique comprend une portée sphérique 6a surmontant l'évidement 7, et une cavité 6b en calotte sphérique aménagée dans la paroi de la pièce 3 formant le fond de cet évidement 7. Cette cavité 6b est aménagée selon une même sphère géométrique que la portée sphérique 6a.

Les reliefs 5 d'ancrage s'étendent, en-dehors de la face 3a, sur l'ensemble de la périphérie de la pièce d'ancrage 3. Ils sont formés par des portions de collerettes saillantes formant des arrêtes terminales vives, propres à pénétrer dans la paroi de l'os spongieux lors d'une impaction de la pièce 3 dans une cavité correspondante pré-aménagée dans l'épiphyse de l'humérus 100.

Ainsi que le montre la figure 3, cette cavité osseuse est aménagée de telle sorte que la pièce 3 puisse être étroitement insérée en elle, avec impaction en fin d'insertion de manière à faire pénétrer les reliefs d'ancrage 5 dans l'os spongieux. En position de complète insertion, la pièce 3 épouse la paroi délimitant cette cavité, et sa face 3a affleure avec la corticale proximale avoisinante.

La pièce articulaire sphérique 2 est alors insérée entre la pièce 3 et la surface glénoïde formée par l'omoplate 101, étant reçue par le siège sphérique 6a, 6b avec calage par rapport à ce siège, c'est-à-dire sans possibilité de roulement de cette pièce 2 par rapport à la pièce d'ancrage 3.

Les parois de la pièce 3 délimitant la portée sphérique 6a et la cavité 6b sont lisses, de même que la paroi de la pièce articulaire 2, de sorte qu'un glissement de cette pièce articulaire 2 par rapport au siège 6a, 6b est possible.

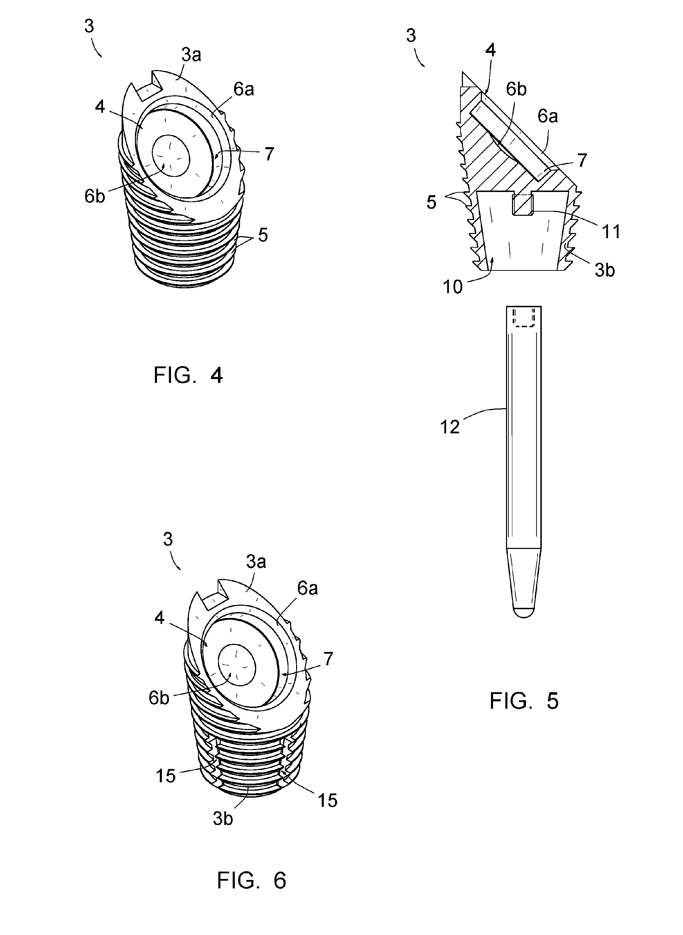

Les figures 4 et 5 montrent une pièce d'ancrage 3 présentant une partie supérieure (celle délimitée par la face 3a) sensiblement identique à la pièce d'ancrage décrite plus haut, avec une cavité proximale 4 identique à celle de cette pièce décrite précédemment. La pièce 3 représentée sur ces figures 4 et 5 a une hauteur plus importante que celle de la pièce d'ancrage décrite précédemment, étant destinée à un ancrage osseux non seulement épiphysaire mais également métaphysaire.

Il apparaît sur la figure 5 que cette pièce 3 comprend une cavité distale 10 débouchant dans son extrémité distale, délimitée par sa paroi périphérique distale 3b. Cette cavité 10 forme un réceptacle à greffon, permettant un ancrage de la pièce 3 à l'humérus 100 par croissance des cellules osseuses dans ce greffon. Compte tenu de la forme conique de la pièce 3, la cavité 10 est en contre-dépouille, assurant une rétention du greffon en elle puis un ancrage de la pièce 3 à la partie osseuse ultérieurement formée par ce greffon, une fois ladite croissance osseuse accomplie.

Il apparaît également sur la figure 5 que la pièce d'ancrage 3 comprend un plot fileté 11 au niveau de sa paroi formant le fond de la cavité 10, ce plot 11 constituant un moyen de liaison à une tige médullaire 12, qui présente un alésage taraudé proximal correspondant.

La tige médullaire 12 permet d'assurer un parfait ancrage de la pièce 3 à l'humérus 100 lorsque la mauvaise qualité de l'os épiphysaire et métaphysaire le requiert.

Les figures 6 à 9 montrent une autre pièce d'ancrage 3 très similaire à celle montrée sur les figures 4 et 5 mais comprenant quatre fentes 15 aménagées au travers de sa paroi périphérique distale 3b, à quatre-vingt-dix degrés les unes des autres. Ces fentes 15 permettent de conférer à cette paroi périphérique distale 3b une souplesse radiale, favorable à la réalisation d'un parfait ancrage distal de la pièce 3 à l'humérus 100. Elles permettent également une croissance des cellules osseuses au travers d'elles, pour une parfaite colonisation par ces cellules du greffon contenu dans la cavité 10.

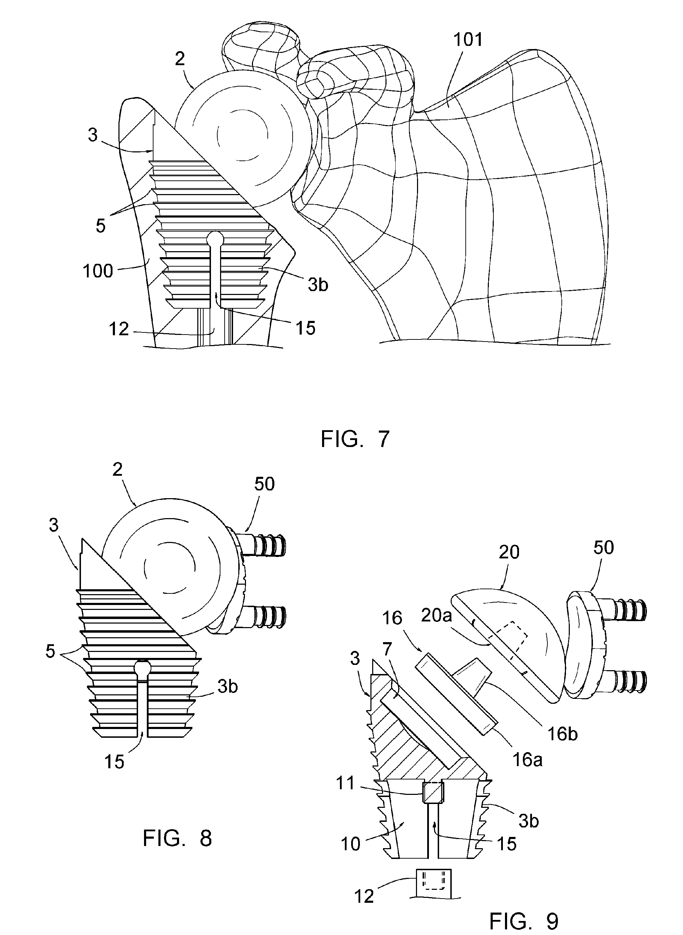

En référence aux figures 7 et 8, il apparaît que la pièce articulaire sphérique 2 peut coopérer soit directement avec la cavité glénoïde formée par l'omoplate 101, soit avec un implant de glène 50, destiné à être mis en place sur l'omoplate 101. De manière bien connue en soi, cet implant 50 comprend un plateau formant la surface d'articulation glénoïde et des plots permettant son ancrage à l'omoplate 101.

La figure 9 montre que l'évidement 7 est apte à recevoir une pièce de montage 16 dont une partie de base 16a comprend une paroi périphérique faiblement conique, adaptée à être insérée jusqu'à coincement dans l'évidement 7. La pièce de montage 16 comprend également un plot conique 16b à faible pente, apte à être engagé jusqu'à coincement dans une cavité correspondante 20a que comprend une tête d'articulation 20 en forme de calotte sphérique. Cette tête 20 forme une surface articulaire propre à coopérer avec l'implant de glène 50.

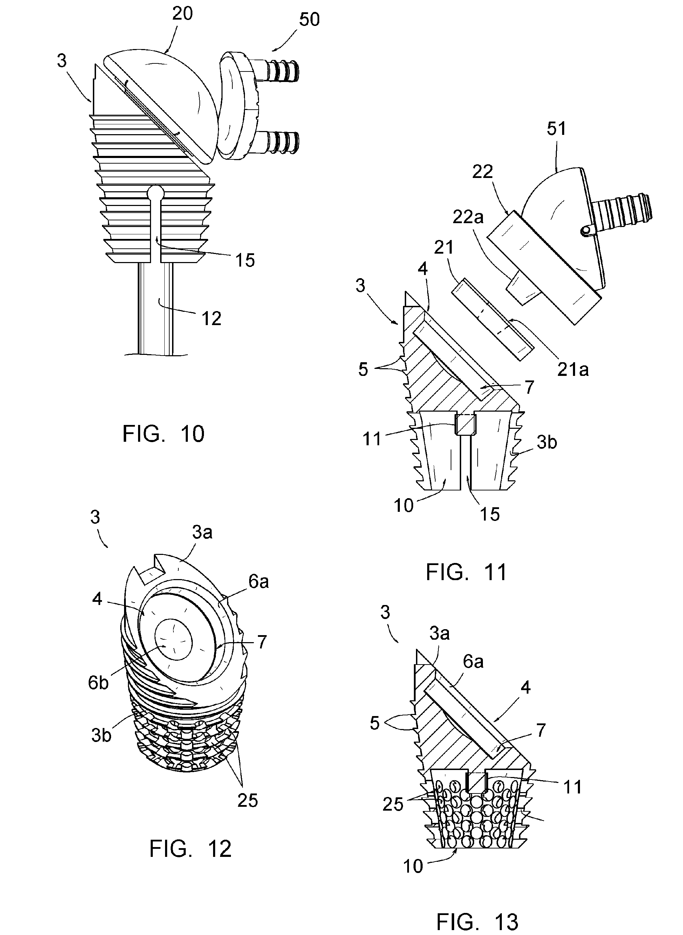

La pièce 16 permet ainsi le montage de la tête 20 sur la pièce d'ancrage 3, pour la constitution d'une prothèse d'épaule dite "anatomique", cf. figure 10.

En référence à la figure 11, il apparaît que, de la même manière, une pièce de montage 21 peut être utilisée pour le montage sur la pièce 3 d'une pièce articulaire 22 à surface d'articulation concave, apte à coopérer avec un implant d'omoplate 51 dit "glènosphère", formant une surface d'articulation arrondie, pour la constitution d'une prothèse dite "inversée". Dans ce cas, la pièce 21 comprend une cavité conique 21a à faible pente aménagée au travers d'elle, et ladite pièce articulaire 22 comprend un plot correspondant 22a, apte à être inséré et coincé dans cette cavité 21 a pour son assemblage à la pièce 3.

Les figures 12 et 13 montrent quant à elles que ladite paroi périphérique distale 3b peut comprendre une pluralité de trous 25 aménagés au travers d'elle, permettant de larges surfaces par lesquelles des cellules osseuses en croissance peuvent coloniser un greffon inséré dans la cavité distale 10.

Ainsi que cela apparaît qui précède, l'invention fournit un implant huméral de prothèse d'épaule ayant pour avantages déterminants de :
- permettre l'utilisation d'une pièce d'ancrage 3 pouvant être ancrée uniquement, ou essentiellement, à la partie épiphysaire et/ou à la partie métaphysaire de l'os, grâce au calage de la pièce articulaire sphérique 2 par rapport à la pièce d'ancrage 3 ;
- ne pas requérir un travail important d'ablation d'os pour sa mise en place, et donc de préserver le capital osseux du patient ;
- éliminer, lorsque la pièce articulaire sphérique 2 est utilisée, un réglage de la position de la pièce articulaire par rapport à la pièce d'ancrage ;
- permettre un parfait fonctionnement de l'articulation reconstruite ;
- permettre la constitution facile et rapide, au besoin en per opératoire, et sans changer substantiellement de technique de pose, de plusieurs types d'implants huméraux.

L'invention a été décrite ci-dessus en référence à une forme de réalisation fournie à titre d'exemple. Il va de soi qu'elle n'est pas limitée à cette forme de réalisation mais qu'elle s'étend à toutes les autres formes de réalisation couvertes par les revendications ci-annexées.

## Revendications

1. Implant huméral (1) de prothèse d'épaule, comprenant une pièce articulaire sphérique (2) et une pièce (3) d'ancrage à l'humérus (100), formant une cavité proximale (4) de réception de cette pièce articulaire sphérique (2) ;
**caractérisé en ce que :**
- ladite cavité proximale (4) est sous forme d'un siège sphérique (6a, 6b) ajusté à la courbure de ladite pièce articulaire sphérique (2) et délimité par un bord périphérique marqué, apte à recevoir cette pièce articulaire sphérique (2) avec calage de cette pièce articulaire par rapport à ce siège, c'est-à-dire de telle sorte que le centre de la pièce articulaire sphérique (2) n'ait pas de possibilité de se déplacer par rapport à la pièce d'ancrage (3) lors du jeu articulaire de la prothèse ; et
- la pièce d'ancrage (3) a une hauteur telle qu'elle est destinée à être implantée dans la partie épiphysaire ou métaphysaire de l'os (100), et comprend des reliefs (5) d'ancrage à l'os.

2. Implant (1) selon la revendication 1, **caractérisé en ce que** la paroi de la pièce d'ancrage délimitant ledit siège sphérique (6a, 6b), ou la paroi de la pièce articulaire sphérique (2), ou les deux, sont lisses de manière à autoriser une possibilité de glissement de la pièce articulaire (2) par rapport à ce siège sphérique (6a, 6b).

3. Implant (1) selon la revendication 1 ou la revendication 2, **caractérisé en ce que** la pièce d'ancrage (3) a une forme conique ou tronconique.

4. Implant (1) selon l'une des revendications 1 à 3, **caractérisé en ce que** les reliefs d'ancrage (5) s'étendent sur au moins une large portion de la périphérie de la pièce d'ancrage (3).

5. Implant (1) selon l'une des revendications 1 à 4, **caractérisé en ce que** la pièce d'ancrage (3) comprend une cavité distale (10) débouchant dans son extrémité distale, délimitée par la paroi périphérique distale (3b) de cette pièce d'ancrage (3).

6. Implant (1) selon la revendication 5, **caractérisé en ce que** ladite paroi périphérique distale (3b) comprend des ouvertures (25) la traversant.

7. Implant (1) selon la revendication 6 ou la revendication 7, **caractérisé en ce que** ladite paroi périphérique distale (3b) comprend au moins une fente (15) la traversant et débouchant dans le bord distal de cette paroi périphérique distale (3b).

8. Implant (1) selon la revendication 5 ou la revendication 6, **caractérisé en ce que** la pièce d'ancrage (3) comprend, au niveau de sa partie distale, un moyen (11) de liaison à une tige médullaire (12).

9. Implant (1) selon la revendication 5 et la revendication 8, **caractérisé en ce que** ledit moyen de liaison est aménagé au niveau de la paroi de la pièce d'ancrage (3) délimitant le fond de ladite cavité distale (10).

10. Ensemble d'éléments assemblables pour la constitution de l'implant huméral (1) de prothèse d'épaule selon l'une des revendications 1 à 9, **caractérisé en ce qu'il** comprend :
- au moins une pièce articulaire sphérique (2) telle que précitée ;
- au moins une pièce d'ancrage (3) telle que précitée, comprenant, adjacent audit siège sphérique (6a, 6b), un évidement proximal (7) faiblement conique, apte à recevoir, avec coincement, une pièce (16, 21) à paroi périphérique faiblement conique correspondante, permettant le montage d'une pièce articulaire (20, 22) non sphérique formant une surface articulaire humérale prothétique ;
- au moins une pièce de montage (16, 21) telle que précitée ; et
- au moins une pièce articulaire non sphérique (20, 22) telle que précitée.

11. Ensemble selon la revendication 10, **caractérisé en ce qu'il** comprend plusieurs pièces articulaires sphériques (2), de diamètres différents.

12. Ensemble selon la revendication 10 ou la revendication 11, **caractérisé en ce que** ledit siège sphérique comprend une portée sphérique (6a) surmontant ledit évidement proximal faiblement conique (7), et une cavité (6b) en calotte sphérique aménagée dans le fond dudit évidement proximal faiblement conique (7), cette cavité (6b) en calotte sphérique étant aménagée selon une même sphère géométrique que ladite portée sphérique (6a).

13. Ensemble selon l'une des revendications 10 à 12, **caractérisé en ce qu'il** comprend plusieurs pièces d'ancrage (3), à savoir au moins une pièce d'ancrage (3) de hauteur telle qu'elle est destinée à être ancrée uniquement à la zone épiphysaire de l'os (100) et au moins une pièce d'ancrage (3) de hauteur telle qu'elle est destinée à être ancrée à la zone épiphysaire et à la zone métaphysaire de l'os.
